(19) Europäisches Patentamt
European Patent Office
Office européen des brevets

(11) Veröffentlichungsnummer : **0 297 391 B1**

# (12) EUROPÄISCHE PATENTSCHRIFT

(45) Veröffentlichungstag der Patentschrift :
**02.05.91 Patentblatt 91/18**

(51) Int. Cl.$^5$ : **C07C 37/52,** C07C 39/19, C07C 39/23

(21) Anmeldenummer : **88109829.7**

(22) Anmeldetag : **21.06.88**

## (54) Verfahren zur Herstellung von monomeren Alkenylphenolen aus Dihydroxydiphenylalkanen.

(30) Priorität : **02.07.87 DE 3721853**

(43) Veröffentlichungstag der Anmeldung :
**04.01.89 Patentblatt 89/01**

(45) Bekanntmachung des Hinweises auf die Patenterteilung :
**02.05.91 Patentblatt 91/18**

(84) Benannte Vertragsstaaten :
**DE FR GB IT**

(56) Entgegenhaltungen :
**DE-A- 2 932 954**
**DE-A- 2 932 959**

(56) Entgegenhaltungen :
**DE-B- 1 235 894**
**US-A- 4 594 459**
**PATENT ABSTRACTS OF JAPAN, unexamined applications, C field, vol. 4, no. 85, June 18, 1980; THE PATENT OFFICE JAPANESE GOVERNMENT, page 75 C 15**

(73) Patentinhaber : **BAYER AG**
**W-5090 Leverkusen 1 Bayerwerk (DE)**

(72) Erfinder : **Dujardin, Ralf, Dr.**
**Bodelschwinghstrasse 18**
**W-4150 Krefeld (DE)**
Erfinder : **Ebert, Wolfgang, Dr.**
**Dörperhofstrasse 31**
**W-4150 Krefeld (DE)**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Jouve, 18, rue Saint-Denis, 75001 PARIS

## Beschreibung

Die Erfindung betrifft ein Verfahren zur Herstellung von monomeren Alkenylphenolen aus Dihydroxydiphenylalkanen durch thermische Spaltung in bestimmten Lösungsmitteln.

Es ist bekannt, daß Dihydroxydiphenylalkane thermisch in Anwesenheit eines basischen Katalysators zu Phenol und Alkenylphenol gespalten werden können.

Aus der GB-PS 905 994 ist bekannt, Dihydroxydiphenylpropan mit Alkali unter vermindertem Druck in der Schmelze zu spalten. Dabei destilliert das Spaltungsprodukt ab. Bei diesem Verfahren wird die Verdampfung von Dihydroxydiphenylpropan mit Hilfe eines Verdampfers vom Flüssigkeitsfilm-Typ bei 260°C durchgeführt. Der Dampf wird dann durch ein Katalysatorbett von Natronkalk geleitet, um in der Gasphase zersetzt zu werden.

Auch sind Verfahren bekannt, bei denen ein unter den Spaltungsbedingungen nicht flüchtiges, inertes Reaktionsmedium verwendet wird. So ist beispielsweise aus der DE-OS 29 32 954 ein Verfahren zur basisch katalysierten Spaltung von Dihydroxydiphenylalkanen bekannt, bei dem man kontinuierlich geschmolzenes Dihydroxydiphenylalkan bei erhöhter Temperatur in ein Reaktionsmedium einbringt das den basischen Katalysator enthält, und woraus dann unter vermindertem Druck die abgespaltenen Alkenylphenole kontinuierlich abdestilliert werden.

Ein Nachteil der bekannten Methoden ist, daß das erhaltene Spaltprodukt neben den monomeren Alkenylphenolen hauptsächlich dimeres Alkenylphenol und Phenol enthält (z.B. DE-OS 29 32 954). Dadurch sind aufwendige Reinigungsoperationen, z.B. Destillationen oder Umkristallisationen notwendig, um das Phenol abzutrennen (z.B. DE-PS 12 35 894). Anschließend muß das hauptsächlich aus dimerem Alkenylphenol bestehende Rohgemisch nochmals thermisch gespalten werden, um reines monomeres Alkenylphenol zu erhalten.

Diese aufwendige, kostenintensive und zeitraubende Reinigungsoperation vermindern zusätzlich die Ausbeute an monomerem Alkenylphenol.

Es wurde nun gefunden, daß man einfacher und mit besseren Ausbeuten Alkenylphenole durch Spaltung von Alkylidenbisphenolen herstellen kann, wenn man die Spaltung in Gegenwart spezieller Lösungsmittel durchführt.

Verfahren zur Herstellung von monomeren Alkenylphenolen aus Dihydroxidiphenylakanen der Formel (I)

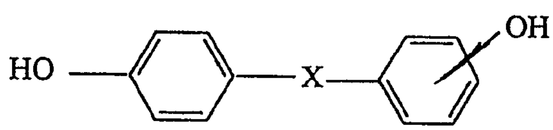

(I),

in welcher

X ein geminaler Alkylidenrest mit 2 bis 12 C-Atomen, ein geminaler Cycloalkyliden-Rest mit 5 bis 7C-Atomen oder ein geminaler Aralkyliden-Rest mit 8 bis 12-C-Atomen ist, in Anwesenheit basischer Katalysatoren, dadurch gekennzeichnet, daß man kontinuierlich eine Lösung aus Dihydroxidiphenylalkan der Formel (I) und einem mit Wasser mischbaren inerten hochsiedenden organischen Lösungsmittel, dessen Siedepunkt bei Normaldruck bis 350°C beträgt,in ein, den basischen Katalysator enthaltendes, organisches Medium bei vermindertem Druck und einer Temperatur von 150 bis 250°C so zuführt, daß sich das Anfangsvolumen des vorgelegten Reaktionsmediums um bis zu 10 Vol-% vergrößert und die Spaltprodukte kontinuierlich zusammen mit dem Lösungsmittel aus dem Reaktionssystem in eine Wasser enthaltende Vorlage abdestillieren, aus der dann das monomere Alkenylphenol isoliert wird und das als Nebenprodukt entstandene Phenol und das Lösungsmittel in der wäßrigen Phase gelöst bleiben.

Beim erfindungsgemäßen Verfahren werden geminale Dihydroxydiphenylalkane, vorzugsweise kernunsubstituierte Dihydroxydiphenylderivate von aliphatischen oder alicyclischen Kohlenwasserstoffen eingesetzt, die in ein Alkenylphenol und Phenol gespalten werden können.

Beispiele erfindungsgemäß verwendbarer Dihydroxydiphenylalkane der Formel (I) sind 2,2-(4,4′-Dihydroxydiphenyl)-propan, 2-(4-Hydroxyphenyl)-2-(2′-hydroxyphenyl)-propan, 1,1-(4,4′-Dihydroxydiphenyl)-ethan, 1,1(4,4′-Dihydroxydiphenyl)-propan, 1,1-(4,4′-Dihydroxydiphenyl)-butan, 2,2-(4,4′-Dihydroxydiphenyl)-butan, 2,2-(4,4′-Dihydroxydiphenyl)-3-methylbutan, 1,1-(4,4′-Dihydroxydiphenyl)-2-methylpropan, 2,2-(4,4′-Dihydroxydiphenyl)-pentan, 3,3-(4,4′-Dihydroxydiphenyl)-pentan, 2,2-(4,4′-Dihydroxydiphenyl)-4-methylpentan, 4,4-(4,4′-Dihydroxydiphenyl)-heptan, 1,1-(4,4′-Dihydroxydiphenyl)-cyclohexan, 1,1-(4,4′-Dihydroxydiphenyl) -1-phenylethan. Bevorzugt ist 2,2-(4,4′-Dihydroxydiphenyl)-propan.

Erfindungsgemäß können als Ausgangsmaterial nicht nur reine Dihydroxydiphenylalkane verwendet werden, sondern auch Rohprodukte wie sie z.B. bei der technischen Herstellung der Dihydroxydiphenylalkane aus

Phenolen und Ketonen anfallen. Das erfindungsgemäße Verfahren wird bei einer Temperatur von 150 bis 250°C durchgeführt. Bevorzugt sind Temperaturen von 200 bis 24O°C.

Bei der erfindungsgemäßen Verfahrensweise wird das Spaltungsprodukt rasch aus dem Reaktionssystem abdestilliert bei einem Druck von 1.000 bis 15.000 Pa.

Für die Durchführung des erfindungsgemäßen Verfahrens werden inerte, hochsiedende organische Lösungomittel verwendet. Deren Siedepunkt beträgt unter Normaldruck bis 350°C. Sie sollten vorteilhaft die Dihydroxydiphenylalkane bei Temperaturen unterhalb von 150°C lösen können. Die Lösungs-(Verdünnungs-)mittel sind vorzugsweise mit Wasser mischbar.

Beispiele für organische Lösungsmittel sind oligomere 1,2-$C_2$-$C_4$-Alkylidenglykole wie Diethylenglykol, Dipropylenglykol, Triethylenglykol und Tripropylenglykol ; Diund Trialkanolamine, z.B. Diethanolamin, Diisopropanolamin, Triethanolamin und Triisopropanolamin ; N-Alkyllactame, z.B. N-Methylpyrrolidon und N-Methyl-C-caprolactam u.s.w. Bevorzugt sind Diethylenglykol und Triethylenglykol.

Bei dem erfindungsgemäßen Verfahren werden basische Katalysatoren verwendet, z.B. Oxide, Mydroxide oder Carbonate von Alkalimetallen oder Erdalkalimetallen wie Natriumhydroxid, Kaliumhydroxid, Natriumcarbonat, Kaliumcarbonat, Calciumoxid und Calciumhydroxid ; Alkalimetallsalze von Phenolen, wie Natriumphenolat, oder ein Natriumsalz eines Kondensationsproduktes gebildet aus Phenol oder Kresol und Aceton (z.B. Bisphenol A) und Alkalimetallsalze von schwach sauren Fettsäuren, wie Natriumacetat u.s.w. Die Menge des basischen Katalysators beträgt 0,01 bis 5 Gew.-%, bezogen auf das Gewicht des eingesetzten Lösungsmittels.

Die erfindungsgemäße Spaltungsreaktion wird durch Zuführung des Reaktionsmediums (Lösungsmittels) und des basischen Katalysators in einen Reaktor, der mit einem Beschickungseinlaß für die Ausgangsmaterial, einem Destillationsauslaß für die Spaltungsprodukte, einem Thermometer und gegebenenfalls einer Rühr–(oder Misch-) vorrichtung ausgestattet ist, so durchgeführt, daß das Reaktionsmedium, das den basischen Katalysator enthält, bei erhöhter Temperatur unter vermindertem Druck von 1.000 bis 15.000 Pa vorgelegt wird und das Dihydroxydiphenylalkan, gelöst im Lösungsmittel, zugeführt wird.

Das Dihydroxydiphenylalkan wird dann in Ahlkenylphenol und Phenol gespalten, wobei die Spaltprodukte mit einem Teil des Lösungsmittels verdampft werden. Dieser Dampf wird rasch aus dem Reaktionssystem in eine mit 5 bis 40°C kaltem wasser beschickte Vorlage abdestilliert. Dabei trennen sich die Spaltprodukte in zwei Phasen auf. Die eine enthält das monomere Alkenylphenol und gegebenenfalls geringe Mengen Phenol, die andere, wäßrige Phase, das gebildete Phenol und das verwendete Lösungsmittel.

Die Alkenylphenole können in feinkristalliner Form oder flüssig anfallen. Feste Alkenylphenole können leicht durch Filtration abgetrennt werden, flüssige durch Phasentrennung.

Für den Fall, daß das Alkenylphenol noch Phenol enthält, erfolgt die Reinigung nach bekannten Methoden.

Die Menge an Wasser in der Vorlage beträgt erfindungsgemäß je nach hergestelltem Alkenylphenol das ein – bis dreifache der Gesamtmenge an eingesetzter Dihydroxydiphenylalkan-Lösung.

Das erfindungsgemäße Verfahren einschließlich der Isolierung der monomeren Alkenylphenole kann kontinuierlich und diskontinuierlich durchgeführt werden.

Bei dem erfindungsgemäßen Verfahren erfolgt die kontinuierliche Zudosierung der Dihydroxydiphenylalkan-Lösung zum vorgelegten Reaktionsmedium so, daß sich das Volumen des vorgelegten Reaktionsmediums um bis zu 10 % erhöht. Vorzugsweise wird die Volumenzunahme des vorgelegten Reaktionsmediums im Bereich von 2 bis 5 Vol-% gehalten.

Die erfindungsgemäße Umsetzung wird unter Durchmischung, vorzugsweise durch Rühren, auch des vorgelegten Wassers in der Vorlage, durchgeführt.

Das erfindungsgemäße Verfahren wird vorzugsweise unter Inertgas wie Stickstoff, Helium oder Argon durchgeführt.

Die monomeren Alkenylphenole werden erfindungsgemäß in hoher Reinheit und in sehr guten Ausbeuten erhalten. Eine weitere Reinigung ist in den meisten Fällen nicht unbedingt erforderlich.

In den Beispielen sind die Prozentangaben ruf das Gewicht bezogen.

Beispeil 1

Man beschickte einen Reaktor (1 l), der mit einem Thermometer, einem Beschickungseinlaß für das Ausgangsmaterial, einem Destillationseinlaß für die Spaltungsprodukte ausgestattet war, mit 250 g Triethylenglykol und 1 g Natriumhydroxid und leitete anschließend 10 Min. Stickstoff durch das Reaktionsmedium. Man hielt die Temperatur des Triethylenglykols bei 230°C und das Innere des Reaktors bei einem Druck von 2666 Pa. Man führte kontinuierlich eine 100°C warme Lösung aus 500 g 2,2-Di-(4-hydroxyphenyl)-propan (im weiteren Text als Bisphenol A bezeichnet) und 450 g Triethylenglykol über den Beschickungseinlaß mit einer Geschwindigkeit von 475 g/h in das aufgeheizte Triethylenglykol. Die aus dem Reaktor abdestillierende Mischung aus Triethylenglykol, p-Isopropenylphenol und Phenol wurde unter Rühren in der mit 1,5 l 20°C kaltem Wasser

beschickten Vorlage (Kapazität 3 l) aufgefangen, wobei das p-Isopropenylphenol als weiBer feinkristalliner Niederschlag ausfiel, der anschließend durch Filtration isoliert wurde.

Die Reaktion lieferte in zwei Stunden aus 500 g Bisphenol A nach dem Trocknen 279 g monomeres p-Isopropenylphenol mit einer Reinheit von größer 99 % (bestimmt durch H-NMR).

Beispiel 2

Man beschickte den gleichen Reaktor wie in Beispiel 1 beschrieben mit 100 g Triethanolamin und 1 g Natriumhydroxid. Man spaltete unter den gleichen Bedingungen wie in Beispiel 1 angegeben eine Lösung aus 500 g Bisphenol A und 500 g Triethanolamin ab. Aus 500 g Bisphenol A wurden 279 g monomeres p-Isopropenylphenol mit einer Reinheit von 99% innerhalb von 8 Stunden erhalten.

Beispiel 3

Man beschickte den gleichen Reaktor wie in Beispiel 1 beschrieben mit 100 g Triethylenglykol und 0,1 g Natriumhydroxid und leitete anschließend 10 Min. Stickstoff durch das Reaktionsmedium. Man führte kontinuierlich eine Lösung aus 1000 g 1,1-Bis-(4-hydroxyphenyl)cyclohexan (im weiteren Text als Bisphenol Z bezeichnet) und 1150 g Triethylonglykol über den Beschickungseinlaß mit einer Geschwindigkeit von 500 g/h in das aufgeheizte Triethylenglykol.

Die Isolierung dos 1-(4-Hydroxyphonyl)-1-cyclohoxens erfolgte analog wie für das p-Isopropenylphonol im Beispiel 1 beschrieben. Aus 1000 g Bisphenol Z wurden 641 g monomeres 1-(4-Hydroxyphenyl)-1-cyclohexen mit einer Reinheit von 99% erhalten.

## Ansprüche

1. Verfahren zur Herstellung von monomeren Alkenylphenolen aus Dihydroxidiphenylalkanen der Formel (I)

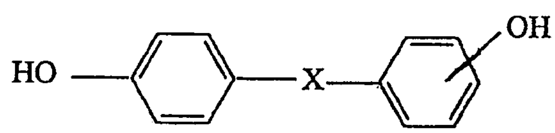

(I),

in welcher

ein geminaler Alkylidenrest mit 2 bis 12 C-Atomen, ein geminaler Cycloalkyliden-Rest mit 5 bis 7 C-Atomen oder ein germinaler Aralkyliden-Rest mit 8 bis 12-C-Atomen ist, in Anwesenheit basischer Katalysatoren, dadurch gekennzeichnet, daß man kontinuierlich eine Lösung aus Dihydroxidiphenylalkan der Formel (I) und einem mit Wasser mischbaren inerten hochsiedenden organischen Lösungsmittel, dessen Siedepunkt bei Normaldruck bis 350°C beträgt, in ein, den basischen Katalysator enthaltendes, organisches Medium bei vermindertem Druck und einer Temperatur von 150 bis 250°C so zuführt, daß sich das Anfangsvolumen des vorgelegten Reaktionsmediums um bis zu 10 Vol-% vergrößert und die Spaltprodukte kontinuierlich zusammen mit dem Lösungsmittel aus dem Reaktionssystem in eine Wasser enthaltende Vorlage abdestillieren, aus der dann das monomere Alkenylphenol isoliert wird und das als Nebenprodukt entstandene Phenol und das Lösungsmittel in der wäßrigen Phase gelöst bleiben.

2. Verfähren gemäß Anspruch 1, dadurch gekennzeichnet, daß als Dihydroxydiphenylalkan 2,2-Bis-(4-hydroxyphenyl)-propan verwendet wird,

3. Verfahren nach Anspruch 1, dadurch gekennzeichnet, daß das Verfahren unter Inertgas ausgeführt wird.

## Claims

1. A process for the production of monomeric alkenyl phenols from dihydroxydiphenyl alkanes corresponding to formula (I)

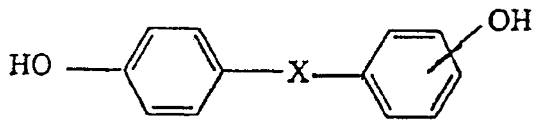

(I),

in which

X is a geminal $C_{2-12}$ alkylidene group, a geminal $C_{5-7}$cycloalkylidene group or a geminal $C_{8-12}$ aralkylidene group, in the presence of basic catalysts, characterized in that a solution of dihydroxydiphenyl alkane corresponding to formula (I) and a water-miscible inert, high-boiling organic solvent, which has a boiling point under normal pressure of up to 350°C, is continuously introduced into an organic reaction medium containing the basic catalyst under reduced pressure and at a temperature of 150 to 250°C at such a rate that the initial volume of the reaction medium is increased by up to 10% by volume and the cleavage products are continuously distilled off together with the solvent from the reaction system into a water-filled receiver from which the monomeric alkenyl phenol is then isolated and the phenol formed as secondary product and the solvent remain dissolved in the aqueous phase.

2. A process as claimed in claim 1, characterized in that 2,2-bis-(4-hydroxyphenyl)-propane is used as the dihydroxydiphenyl alkane.

3. A process as claimed in claim 1, characterized in that the process is carried out in an inert gas.

**Revendications**

1. Procédé pour la fabrication d'alcénylphénols monomères à partir de dihydroxydiphénylalcanes de formule (I)

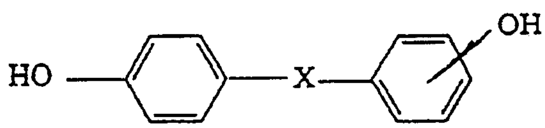

(I),

dans laquelle,

X est un reste alkylidène géminé en $C_2$-$C_{12}$, un reste cycloalkylidène géminé en $C_5$ -$C_7$ ou un reste arylalkylidène géminé en $C_8$-$C_{12}$,

en présence de catalyseurs basiques, caractérisé en ce que l'on envoie en continu une solution du dihydroxydiphénylalcane de formule (I) et d'un solvant organique inerte de haut point d'ébullition, miscible avec l'eau, dont le point d'ébullition sous pression normale va jusqu'à 350°C, sous pression réduite à une température de 150 à 250°C dans un milieu organique contenant le catalyseur basique, de telle sorte que l'augmentation de volume du milieu de réaction initial chargé préalablement aille jusqu'à 10 % en volume et que les produits de dissociation soient distillés du système de réaction en même temps que le solvant dans un ballon récepteur contenant de l'eau, à partir duquel l'alcénylphénol monomère est ensuite isolé et le phénol formé comme sous-produit et le solvant restent dissous dans la phase aqueuse.

2. Procédé selon la revendication 1, caractérisé en ce que l'on utilise comme dihydroxydiphénylalcane le 2,2-bis-(4-hydroxyphényl)-propane.

3. Procédé selon la revendication 1, caractérisé en ce que le procédé est mis en oeuvre en atmosphère de gaz inerte.